# EUROPÄISCHE PATENTSCHRIFT

(11) **EP 0 376 116 B1**
(45) Veröffentlichungstag und Bekanntmachung des Hinweises auf die Patenterteilung: **05.10.1994**
(21) Anmeldenummer: 89123410.6
(22) Anmeldetag: 19.12.1989
(51) Int. Cl.: G01N 27/82, G01N 33/38

(54) **Verfahren zur Ermittlung der Lage einer Bruchstelle eines Spannstahls**
Method for identifying the position of a crack in reinforcing steel
Procédé pour identifier la position d'une cassure dans une armature en acier

(30) Priorität: 29.12.1988 DE 3844216; 14.07.1989 DE 3923377
(43) Veröffentlichungstag der Anmeldung: 04.07.1990
(73) Patentinhaber: HOCHTIEF AKTIENGESELLSCHAFT VORM. GEBR. HELFMANN, 45128 Essen (DE)
(72) Erfinder: Hillemeier, Bernd, Dr.-Ing., D-6200 Wiesbaden (DE); Flohrer, Claus, Dipl.-Ing., D-6078 Neu-Isenburg (DE); Schaab, Andreas, Dipl.-Ing. (FH), D-8750 Aschaffenburg (DE)
(74) Vertreter: Andrejewski, Walter

(56) Entgegenhaltungen:
- EP-A- 0 027 368
- EP-A- 0 096 078
- US-A- 4 087 749
- US-A- 4 531 091
- PATENT ABSTRACTS OF JAPAN Band 5, Nr. 138 (P-78)(810),2. September 1982; & JP-A-56073344

## Beschreibung

Die Erfindung betrifft ein Verfahren zur Ermittlung der Lage einer Bruchstelle eines Spannstahls in einem Spannbetonbauteil, in dessen Beton der Spannstahl bei bekannter Orientierung unmittelbar oder mit dielektrischer Umhüllung eingebettet ist, wobei der zu untersuchende Spannstahl mit Hilfe einer Magnetisierungseinrichtung magnetisiert und das den Spannstahl umgebende Magnetfeld untersucht wird.

Bei den in der Praxis üblichen Verfahren zur Ermittlung der Lage einer Bruchstelle eines Spannstahls wird das Spannbetonbauteil mit Röntgenstrahlen, Gammastrahlen oder anderen Strahlen der zerstörungsfreien Werkstoffprüfung durchstrahlt. Dadurch können der Verlauf eines Spannstahls und eine Bruchstelle in dem Spannstahl sichtbar gemacht und zur Anzeige gebracht werden. Das ist aufwendig und setzt die Durchstrahlbarkeit, d. h. zumindest zwei zugängliche und einander gegenüberliegende Oberflächen des Spannbetonbauteils, zwischen denen der zu untersuchende Spannstahl angeordnet ist, voraus. Ähnlich liegen die Verhältnisse, wenn man durchstrahlend mit Ultraschall arbeitet. Zwar kennt man in beiden Fällen auch Untersuchungen mit Reflektion, die eine Durchstrahlung nicht erforderlich machen, hier reicht jedoch die Anzeige häufig nicht aus, weil bei Untersuchungen mit reflektierten Strahlen oder Wellen das Auflösungsvermögen sehr viel kleiner ist als bei Durchstrahlung. In der Praxis ist es auch bekannt, den Ohmschen Widerstand eines Spannstahls als Kriterium für einen Spannstahlbruch einzusetzen, da eine Bruchstelle den Ohmschen Widerstand unstetig ansteigen oder praktisch unendlich groß werden läßt. Hierzu ist es jedoch zunächst erforderlich, die Enden des zu untersuchenden Spannstahls freizulegen. Darüber hinaus ist bei einer solchen Untersuchung die Ermittlung der Lage der Bruchstelle in dem Spannbetonbauteil schwierig.

Bei dem bekannten Verfahren, von dem die Erfindung ausgeht (US-PS 45 31 091), wird in einem Verfahrensschritt der Spannstahl durch ein aktives, durch einen Elektromagneten als Magnetisierungseinrichtung erzeugtes Magnetfeld magnetisiert und gleichzeitig wird das dadurch entstehende aktivierte Magnetfeld in der Umgebung des Spannstahls im Bereich der Magnetisierungseinrichtung, nämlich zwischen deren Polen, mit einem Hall-Generator als Magnetisierungsmeßsonde abgefragt. Dabei wird die Vorrichtung mit der Magnetisierungsvorrichtung und der Meßsonde längs des zu untersuchenden Spannstahls an dem Stahlbetonbauteil entlanggeführt. Regelmäßig sind in einem Stahlbetonbauteil die Spannstähle mit quer zu ihrer Erstreckung verlaufenden Stahlbügeln kombiniert. Bei den bekannten Maßnahmen werden auch diese aktiv magnetisiert und bilden auch diese in ihrer Umgebung ein aktiviertes Magnetfeld. Das gilt auch für dem zu untersuchenden Spannstahl benachbarte Spannstähle, und zwar insbesondere dann, wenn diese mit den Stahlbügeln verbunden sind. Diese von den Stahlbügeln und benachbarten Stahlstäben ausgehenden aktivierten Magnetfelder stören im Rahmen der bekannten Maßnahmen die Feststellung einer Bruchstelle in dem zu untersuchenden Spannstahl und machen aufwendige Analysen der Meßergebnisse erforderlich. Auch kann infolge dieser Störungen nicht ausgeschlossen werden, daß eine Bruchstelle übersehen wird.

Demgegenüber liegt der Erfindung die Aufgabe zugrunde, ein Verfahren zur Ermittlung der Lage einer Bruchstelle eines Spannstahls in einem Spannbetonbauteil anzugeben, dessen Meßergebnisse und dessen Untersuchungssicherheit durch die beschriebenen Störungen nicht mehr in störendem Maße beeinträchtigt ist.

Zur Lösung dieser Aufgabe lehrt die Erfindung, daß in einem ersten Verfahrensschritt der Spannstahl lediglich magnetisiert sowie danach die Magnetisierungseinrichtung entfernt wird, daß danach in einem zweiten Verfahrensschritt das Magnetfeld des im Spannstahl nach der Entfernung der Magnetisierungseinrichtung verbleibenden remanenten Magnetismus abgefragt und dabei eine Dipolbildung als Kriterium für die Bruchstelle ermittelt wird. - In der Physik wird die Magnetisierung bekanntlich durch die Magnetisierungskurve dargestellt, die für ferromagnetische Materialien und insbesondere für den Werkstoff Stahl auch als Hysteresis-Schleife bezeichnet wird. Wird ein ferromagnetisches Bauteil von einem fremden äußeren Magnetfeld beeinflußt, so erfährt es eine Magnetisierung, die von der äußeren Feldstärke und im übrigen von Materialparametern abhängt. In einem carthesischen Koordinatensystem wird die fremde äußere Feldstärke im allgemeinen als Abszisse aufgetragen, während die Magnetisierung auf der Ordinate abgebildet wird. Die Magnetisierungskurve zeigt, daß die Magnetisierung von der magnetischen Vorgeschichte des Bauteils abhängt. Wird das fremde äußere Magnetfeld zu Null, so wird die Magnetisierung nicht ohne weiteres gleichzeitig Null. Es bleibt vielmehr ein remanenter Magnetismus, die sogenannte Remanenz. Ihr Maß ist bei aufgenommener Magnetisierungskurve an dem Schnittpunkt der Magnetisierungskuve mit der Ordinatenachse ablesbar. Die Remanenz kann bei Stahl groß sein und ist bei sogenannten Dauermagneten sehr groß. Im Gegensatz zur Remanenz steht das eingangs behandelte, aktivierte Magnetfeld, welches, wie beschrieben, auf ein von Null verschiedenes, zumeist von Null beachtlich verschiedenes fremdes aktives Magnetfeld reagiert. Die Erfindung nutzt das Naturgesetz, daß ein Stabmagnet mit Nordpol und Südpol, wird er quer geteilt, gleichsam in zwei Stabmagnete zerfällt, wobei an der Teilungsstelle ein Nordpol und ein Südpol einander gegenüberstehen. Die Erfindung geht von der Erkenntnis aus, daß an der Bruchstelle eines Spannstahls in einem Spannbetonbauteil eine aus dem remanenten Magnetismus resultierende Polarisierung unschwer mit geeigneten Meßgeräten erfaßt werden kann, weil sich die beiden Pole an der Bruchstelle infolge der dort aufgehobenen mechanischen Zugspannung in dem Spannstahl um ein geringes Maß voneinander entfernen, so daß eine deutliche magnetische Dipolbildung aus dem remanenten Magnetismus eintritt, welche jedenfalls ausreicht, meßtechnisch erfaßt zu werden. Eine dielektrische Umhüllung hindert diese Erfaßbarkeit ebensowenig wie die zuvor duchzuführende Magnetisierung. Überraschenderweise beeinträchtigen in der Umgebung der Bruchstelle eines Spannstahls angeordnete Stahlbügel oder benachbarte Spannstähle die Messung nicht mehr störend.

Im einzelnen bestehen im Rahmen der Erfindung mehrere Möglichkeiten zur Durchführung des erfindungsgemäßen Verfahrens. So kann der Spannstahl mit Hilfe eines Permanentmagneten oder einer Magnetisierungs-Spule magnetisiert werden, die am Spannbetonbauteil längs der Erstreckung des Spannteils entlanggeführt wird. Als Magnetisierungsmeßsonde kann im einfachsten Fall ein Magnet, z. B. eine Kompaßnadel eingesetzt werden, wobei der Ausschlag dieses Magneten bzw. der Kompaßnadel die Bruchstelle anzeigen. Als Magnetisierungsmeßsonde können aber auch eine einwindige oder mehrwindige Detektor-Spule oder eine Hallsonde eingesetzt werden. Dabei besteht die Möglichkeit, den Detektorstrom der Detektor-Spule an einem Kathodenstrahloszillographen oder dergleichen zur Anzeige zu bringen. Die Magnetisierung kann auch mit Hilfe eines Permanentmagneten durchgeführt werden, der längs der Richtung eines Spannstahls über die Oberfläche des Spannbetonbauteils geführt wird.

Im folgenden wird die Erfindung anhand einer lediglich ein Ausführungsbeispiel darstellenden Zeichnung ausführlicher erläutert. Es zeigen in schematischer Darstellung
- Fig. 1: ein Spannbetonbauteil in Form eines Balkens mit eingebetteten Spannstählen,
- Fig. 2: eine graphische Darstellung zu dem Gegenstand der Fig. 1.

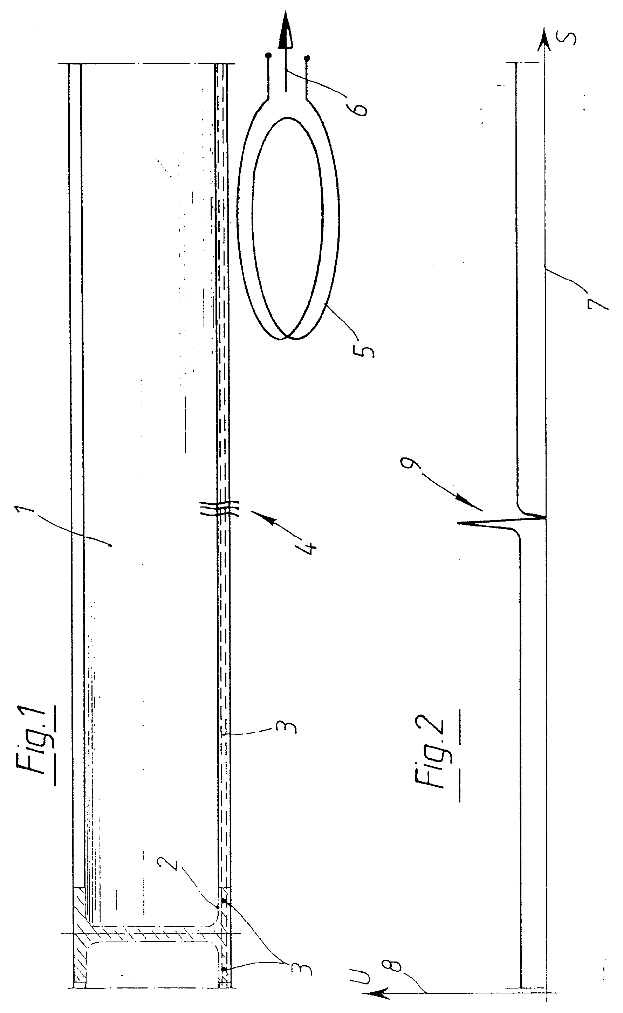

Das Spannbetonbauteil der Fig. 1 hat die Form eines Balkens 1 mit grundsätzlich I-förmigem Querschnitt, wobei im Untergurt 2 zwei Spannstähle 3 einbetoniert sind. Einer dieser Spannstähle mag bei 4 gebrochen sein. In der Fig. 1 erkennt man, daß eine Magnetisierungs-Spule 5 vorgesehen ist, die in Richtung des Pfeiles 6 bewegt wird und die Bruchstelle 4 bereits passiert hat. Sie wird danach entfernt. Im Spannstahl 3 verbleibt der remanente Magnetismus.

In der Fig. 2 ist auf der Abszissenachse 7 die Länge S des Stahlbetonbauteils 1 aufgezeichnet, auf der Ordinatenachse 8 die induzierte Spannung U einer Detektor-Spule die längs des Spannbetonbauteils 1 und damit längs eines zu untersuchenden Spannstahls 3 geführt worden ist und dabei die Bruchstelle 4 des Spannstahls 3 passiert hat. Man erkennt an der Bruchstelle eine deutliche Unstetigkeit 9 in der Anzeige des Induktionsstromes. Sie resultiert aus der beschriebenen Dipolbildung, die auf dem remanenten Magnetismus in dem Spannstahl nach Entfernung der Magnetisierungs-Spule 5 beruht. Eine sehr genaue Ortung der Lage der Bruchstelle 4 ist damit möglich. Ein Stahlbügel in der Nähe der Bruchstelle, der quer zu den Spannstählen 3 verläuft und diese verbindet, würde nicht stören.

## Patentansprüche

1. Verfahren zur Ermittlung der Lage einer Bruchstelle eines Spannstahls in einem Spannbetonbauteil, in dessen Beton der Spannstahl bei bekannter Orientierung unmittelbar oder mit dielektrischer Umhüllung eingebetter ist, wobei der zu untersuchende Spannstahl mit Hilfe einer Magnetisierungseinrichtung magnetisiert und das den Spannstahl umgebende Magnetfeld untersucht wird, **dadurch gekennzeichnet,** daß in einem ersten Verfahrensschritt der Spannstahl lediglich magnetisiert sowie danach die Magnetisierungseinrichtung entfernt wird, daß danach in einem zweiten Verfahrensschritt das Magnetfeld des im Spannstahl nach der Entfernung der Magnetisierungseinrichtung verbleibenden remanenten Magnetismus abgefragt und dabei eine Dipolbildung als Kriterium für die Bruchstelle ermittelt wird.

2. Verfahren nach Anspruch 1, dadurch gekennzeichnet, daß der Spannstahl mit Hilfe eines Permanentmagneten oder einer Magnetisierungs-Spule magnetisiert wird, die am Spannbetonbauteil längs der Erstreckung des Spannstahls entlanggeführt wird.

3. Verfahren nach einem der Ansprüche 1 oder 2, dadurch gekennzeichnet, daß als Magnetisierungsmeßsonde ein Magnet, z. B. eine Kompaßnadel, eingesetzt wird.

4. Verfahren nach einem der Ansprüche 1 oder 2, dadurch gekennzeichnet, daß als Magnetisierungsmeßsonde eine einwindige oder mehrwindige Detektor-Spule eingesetzt wird.

5. Verfahren nach einem der Ansprüche 1 oder 2, dadurch gekennzeichnet, daß als Magnetisierungsmeßsonde ein Hall-Generator eingesetzt wird.

6. Verfahren nach einem der Ansprüche 4 oder 5, dadurch gekennzeichnet, daß der Detektor-Induktionsstrom der Detektor-Spule an einem Kathodenstrahloszillographen zur Anzeige gebracht wird.

## Claims

1. A method of determining the position of a fracture of a prestressing steel element in a prestressed concrete component, in the concrete of which the prestressing steel is embedded with a known orientation directly or with a dielectric cladding, wherein the prestressing steel to be tested is magnetised by means of a magnetisation device and the magnetic field surrounding the prestressing steel is investigated, characterised in that in a first procedural step the prestressing steel is simply magnetised and that thereafter the magnetisation device is removed, that thereafter in a second procedural step the magnetic field of the residual magnetism remaining in the prestressing steel after the removal of the magnetisation device is scanned and in this connection the formation of a dipole is determined as a criterion for the fracture.

2. A method according to claim 1, characterised in that the prestressing steel is magnetised by means of a permanent magnet or a magnetisation coil, which is guided on the prestressed concrete component along the span of the prestressing steel.

3. A method according to either one of claims 1 or 2, characterised in that a magnet, e.g. a compass needle, is used as the magnetisation measuring head.

4. A method according to either one of claims 1 or 2, characterised in that a single-turn or multiple turn detector coil is used as the magnetisation measuring head.

5. A method according to either one of claims 1 or 2, characterised in that a Hall generator is used as the magnetisation measuring head.

6. A method according to either one of claims 4 or 5, characterised in that the detector induction current of the detector coil is caused to display on a cathode ray oscillograph.

## Revendications

1. Procédé visant à déterminer la position d'un point de rupture dans un élément en béton précontraint, béton dans lequel l'acier de précontrainte est noyé dans une disposition connue soit directement soit dans une enveloppe diélectrique; l'acier de contrainte soumis à l'examen étant magnétisé à l'aide d'un dispositif de magnétisation et le champ magnétique enveloppant l'acier de contrainte étant analysé **caractérisé par le fait** que, dans une première étape du procédé, l'acier de contrainte est uniquement magnétisé et que le dispositif de magnétisation est ensuite retiré, et que dans une seconde étape du procédé, le champ magnétique du magnétisme rémanent présent dans l'acier de contrainte est échantillonné après le retrait du dispositif de magnétisation et une génération de dipôle est déterminée en tant que critère du point de rupture.

2. Procédé selon la revendication 1, **caractérisé par le fait** que l'acier de contrainte est magnétisé à l'aide d'un aimant permanent ou d'une bobine à réaimanter qui est placée sur l'élément en béton précontraint, sur toute la longueur de l'acier de contrainte.

3. Procédé selon l'une des revendications 1 ou 2, **caractérisé par le fait** qu'un aimant, une aiguille de boussole p.ex., est utilisé en tant que sonde de mesure d'aimantation.

4. Procédé selon l'une des revendications 1 ou 2, **caractérisé par le fait** qu'une bobine détectrice à une ou plusieurs spires est utilisée en tant que sonde de mesure d'aimantation.

5. Procédé selon l'une des revendications 1 ou 2, **caractérisé par le fait** qu'un générateur Hall est utilisé en tant que sonde de mesure d'aimantation.

6. Procédé selon l'une des revendications 4 ou 5, **caractérisé par le fait** que le courant d'induction détecteur de la bobine détectrice soit affiché sur un oscillographe cathodique.
